# EUROPEAN PATENT APPLICATION

(11) **EP 1 053 761 A1**
(43) Date of publication of application: **22.11.2000**
(21) Application number: 99121140.0
(22) Date of filing: 22.10.1999
(51) Int. Cl.: A61M 5/168

(54) **Multifunction drip unit for infusion equipment**

(30) Priority: 19.05.1999 HU 9901650
(71) Applicant: DISPOMEDICOR Részvénytarsasag, H-4027 Debrecen (HU)
(72) Inventor: Dobos, Janos, 4225 Debrecen (HU); Ladi, Béla, 4027 Debrecen (HU)
(74) Representative: Viering, Jentschura & Partner

(57) **Abstract**

Multifunction drip unit for infusion sets, which consists of a house enclosing a drip chamber suitable for the temporary storage of infusion liquid, a head piece situated at the inlet side of the house equipped with a puncture spike, and a discharge stub extending from the outlet side of the house which can be connected to the infusion tube. A flow control unit (20) is inserted between the discharge stub (15) and the drip chamber (12), the flow control unit (20) has a control area (22) and a cock piece (25) reaching into the control area (20) and equipped with a movable sealing surface (26) there, the control area (22) is connected with the house's (11) drip chamber (12) through the first passage (23) and with the discharge stub (15) through the second passage (24), the sealing surface (26) is placed in the control area (22) so that it covers at least either the first passage (23) or the second passage (24) to a variable extent.

## Description

The subject of the invention is a multifunction drip unit for infusion sets which consists of a house enclosing a drip chamber suitable for the temporary storage of infusion liquid, a head piece situated at the inlet side of the house equipped with a puncture spike, and a discharge stub extending from the outlet side of the house which can be connected to the infusion tube.

Medical institutes, hospitals, ambulance services and other units providing emergency aid use a wide range of infusion and transfusion sets. Drip chambers are important elements of infusion sets used for facilitating the ingestion of different liquids into the body, and their main point is that they are connected between the bag containing the infusion solution and the infusion tube and by this way they make it possible for the liquid content of the bag to be ingested into the circulatory system of the target person in a regulated way.

With the simple drip chambers used presently the rate of the liquid intake cannot be regulated properly. For this reason, in simpler cases, in the interest of realising more precise control, supplementary unit is placed beside the drip chamber containing a roller, a flow control clamp situated on the infusion tube. However, the disadvantage of this solution is that the control range achieved in this way is rather narrow and the continuous maintenance of the set values is not satisfactory.

Another disadvantage is that in the case of using PVC-free sets, which are less harmful to the environment, due to the fact that the materials are less flexible the maintenance of the set values is even worse.

In cases when more precise control is required more complex equipment is used. Patent No. HU T/50.638 relates to a drip chamber suitable for sensing the flow rate. In this solution the rate of the liquid drips is measured with an optically operating sensing device which is fitted to the house of the drip chamber in a special way. Another infusion set combination is described in patent No. HU 187.406.

However, in the case of drip control devices realising fine setting and value maintenance of the control range it is a disadvantage that, because of their difficult construction and larger dimensions, their production is expensive, the devices are difficult to handle and they require more space.

Our aim with the solution according to the invention was to overcome the deficiencies of the drip control devices connected to the known drip chambers and to create a version which has small dimensions, is easy to handle and provides a wide control range and appropriate value maintenance for a long period of time.

The invention is based on the recognition that if the structural elements performing the drip control are not connected to the infusion tube and its cross-section is not changed, but we interfere with the process in the section before the feeding tube and the flow control unit is allocated to the drip chamber or perhaps the control is performed inside it, and in the flow control unit a control unit of a special geometric construction operating differently from the known units is used, then the task can be solved.

In accordance with the set aim, the multifunction drip unit for infusion sets, which consists of a house enclosing a drip chamber suitable for the temporary storage of infusion liquid, a head piece situated at the inlet side of the house equipped with a puncture spike , and a discharge stub extending from the outlet side of the house which can be connected to the infusion tube, is constructed so that a flow control unit is inserted between the discharge stub and the drip chamber, the flow control unit has a control area and a cock piece reaching into the control area and equipped with a movable sealing surface there, the control area is connected with the house's drip chamber through the first passage and with the discharge stub through the second passage, and the sealing surface is placed in the control area so that it covers at least either the first passage or the second passage to a variable extent.

A further feature of the drip unit according to the invention can be that the house and the bordering shell of the flow control unit is formed from one single piece, the drip chamber and the control area are separated from each other with a partition wall, and the first passage is put in the partition wall.

In the case of a possible construction of the drip unit the sealing surface is a cylindrical shell, and the sealing surface is bordered by a spiral shaped confining ridge.

From the aspect of the invention, it is advantageous if the sealing surface of the cock piece is connected to the drive head, and the drive head is placed outside the bordering shell of the flow control unit.

In the case of another different construction of the drip unit either the drive head and the house or the bordering shell is provided with a scale and the other one is provided with markings.

The multifunction drip unit according to the invention has several advantageous features. The most important one of these is that in the course of using the specially constructed and arranged flow control unit the drip count of the liquid to be ingested can be set in a simple way within a wider range and significantly more precisely.

It must also be regarded as an advantage that due to the construction the constancy of the set values in time is also better, and because of the special position of the new version PVC-free sets and tubing can be used without any difficulty.

It can also be regarded as favourable that the construction according to the invention can be produced in a simple way, with traditional tools, its space demand is minimal, and its handling is also simpler than that of the traditional fine-setting devices.

Below the drip unit according to the invention is described with a construction example, on the basis of a drawing. In the drawing
figure 1 is the longitudinal section of the drip chamber according to the invention,
figure 2 is the view of figure 1 taken from the direction of plane II.

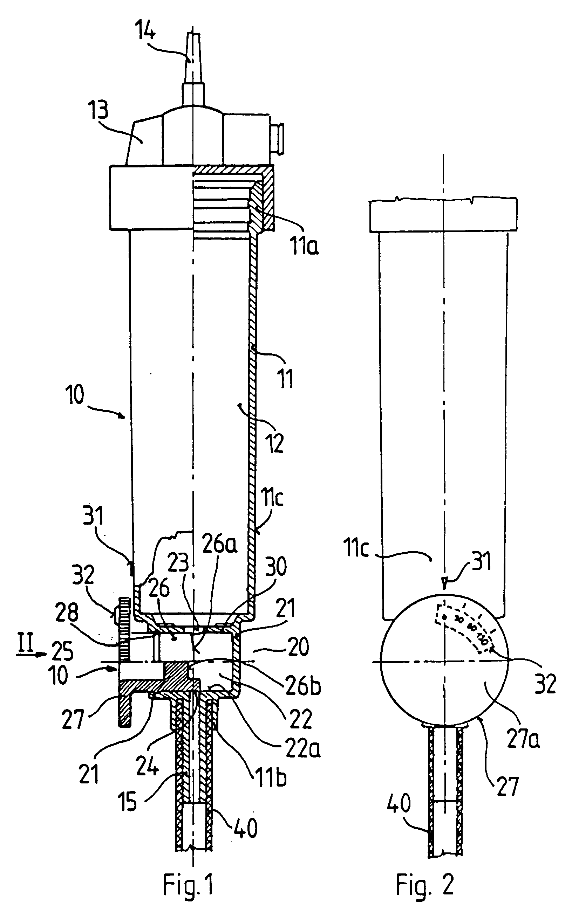

Figures 1 and 2 show a possible construction of the drip unit 10 according to the invention. It can be seen well that the house 11 which holds the drip chamber 12 and which, in our case, is made of transparent plastic, is equipped with a head piece 13 at the inlet side 11a and with a discharge stub 15 at the outlet'side 11b of the house 11. The head piece 13 is equipped with a puncture spike 14 which serves for the connection to the infusion bottle. The infusion tube 40 is connected to the discharge stub 15.

In figure 1 it can also be seen that the house 11 is complemented with a flow control unit 20 which, in this case, is best inserted between the drip chamber 12 and the discharge stub 15 of the house 11. The flow control unit 20 consists of the bordering shell 21, the control area 22 enclosed by the bordering shell 21 and the cock piece 25.

The bordering shell 21 of the flow control unit 20 and the house 11 are made of the same material, the two structural elements make one unit. As a result of this in the case of the present construction of the solution the control area 22 enclosed by the bordering shell 21 and the drip chamber 12 of the house 11 are separated from each other only with a partition wall 30. In the partition wall 30 there is the first passage 23 which makes passage possible between the drip chamber 12 and the control area 22, while liquid flow connection between the control area 22 and the discharge stub 15 is provided by the second passage 24.

The sealing surface 26 of the cock piece 25 belonging to the flow control unit 20 is placed inside the control area 22 and it is equipped with a drive head 27 extending from the control area 22. The sealing surface 26 is constructed so that its external dimensions fit the part of the internal surface 22a control area 22 in the vicinity of the first passage 23 or the second passage 24. In the interest of the simple construction and production technology preferably the internal surface 22a by the bordering the control area 22 should have the shape of a cylindrical shell, and consequently the sealing surface 26 of the cock piece 25 also has the shape of a cylindrical shell.

It can be observed that a clamp 21a is sunk in the internal surface 22a of the control area 22 of the bordering shell 21 and in our case it is a recess running around. At the same time the sealing surface 26 is equipped with another clamp 28 constructed fitting to the first clamp 21a, and in this case it is a protruding rib running around radially on the cylindrical shell shaped sealing surface 26. The first clamp 21a and the other clamp 28 are co-ordinated so that they ensure appropriate locking between the bordering shell 21 and the sealing surface 26 and make it possible to turn around the sealing surface 26 with the help of the drive head 27 and at the same time prevent the cock piece 25 from being pulled out of the control area 22.

The end 26b of the sealing surface 26 on the side of the control area 22 is closed by a spiral shaped confining ridge 26a which is situated at the end 26b of the sealing surface 26 so that when the drive head 27 is turned around the confining ridge 26a drags along the first passage 23 situated in the parting wall 30, and so the state of complete opening and complete locking can be reached in the case of a defined position of the sealing surface 26.

In figure 2 it can be easily seen that the drive head 27 of the cock piece 25 is shaped like a circular plate, and on its external surface 27a there is a scale 32 which shows the quantity of the liquid flowing through the opening resulting from the position of the confining ridge 26a of the sealing surface 26 and the first passage 23 with respect to each other, practically stating the drop count per minute. In the interest of making the scale 32 interpretable and readable there is a sign 31 on the outer surface 11c of the house 11. The function of the sign 31 is to show in which position of the drive head 27 the given mark on the scale 32 situated on the external side 27a of the drive head 27 is authoritative.

It must be pointed out here that the cock piece 25 of the flow control unit 20 can be different from that described here, and the drop count setting can be solved not only by turning the cock piece 25 but also by sliding it. However, simple production technology and handling aspects make the construction of a cylindrical shell shaped control area 22 and sealing surface 26 more favourable.

Obviously another version can also be produced where the bordering shell 21 of the flow control unit 20 is not a part of the house 11, but the whole of the flow control unit 20 is independent from the house 11 and can be fitted to it subsequently. However, even in the case of this version it is a requirement that the flow control unit 20 is inserted between the house 11 and the infusion tube 40.

When using the drip unit according to the invention the puncture spike 14 belonging to the head piece 13 of the house 11 is connected to the infusion bottle not shown in the figures in the usual way. Then the drive head 27 of the cock piece 25 of the flow control unit 20 is turned so that the mark 32 on the scale showing the desired drop count is situated right beside the mark 31 on the outer surface 11c of the house 11. While turning the drive head 27 of the cock piece 25 the sealing surface 26 turns, too. While the sealing surface 26 is turning around always a different part of the confining ridge 26a is set above the first passage 23 ensuring passage between the control area 22 and the drip chamber 12, because in the course of turning the sealing surface 26 the spiral shaped confining ridge 26a changes its position so that depending on the extent of the turning it can move from one edge of the first passage 23, that is from its locked position, to the other edge of the first passage 23, that is to its open position. So by moving the confining ridge 26a of the sealing surface 26 the cross-section of the first passage 23 not covered by the locking surface 26, that is its actual aperture size can be influenced, which makes it possible to maintain the desired value of the drop count per minute.

After setting the aperture size of the first passage 23 the liquid situated in the drip chamber 12 can flow into the control area 22 in accordance with the actual cross-section of the first passage 23, and from there, through the second passage 24 into the discharge stub 15 and into the infusion tube 40 connected to it.

In the meantime the drop count can be reset by turning the drive head 27 of the cock piece 25 again, and after this the actual aperture size of the first passage 23 can be changed as described above. The flow can be stopped by turning the drive head 27 of the cock piece 25 to one of the end positions, while the first passage 23 can be completely opened by turning the drive head 27 of the cock piece 25 to the other end position.

The drip unit according to the invention is ideal for infusion sets, for the fine-setting of the drop count per minute prescribed in the course of the therapy and to maintain the set value for a long period of time.

### List of references

10 drip unit
   11 house
   11a inlet side
   11b outlet side
   11c outer surface
   12 drip chamber
   13 head piece
   14 puncture spike
   15 discharge stub
20 flow control unit
   21 bordering shell
   21a clamp
   22 control area
   22a internal surface
   23 first passage
   24 second passage
   25 cock piece
   26 locking surface
   26a confining ridge
   26b end
   27 drive head
   27a external side
   28 other clamp
30 partition wall
31 sign
32 scale
40 infusion tube

## Claims

1. Multifunction drip unit for infusion sets, which consists of a house enclosing a drip chamber suitable for the temporary storage of infusion liquid, a head piece situated at the inlet side of the house equipped with a puncture spike, and a discharge stub extending from the outlet side of the house which can be connected to the infusion tube, **characterised by** that a flow control unit (20) is inserted between the discharge stub (15) and the drip chamber (12), the flow control unit (20) has a control area (22) and a cock piece (25) reaching into the control area (20) and equipped with a movable sealing surface (26) there, the control area (22) is connected with the house's (11) drip chamber (12) through the first passage (23) and with the discharge stub (15) through the second passage (24), the sealing surface (26) is placed in the control area (22) so that it covers at least either the first passage (23) or the second passage (24) to a variable extent.

2. Multifunction drip unit as in claim 1, **characterised by** that the house (11) and the bordering shell (21) of the flow control unit (20) are constructed from one single piece, the drip chamber (12) and the control area (22) are separated from each other with a partition wall (30), and the first passage (23) is put in the partition wall (30).

3. Multifunction drip unit as in claim 1 or 2, **characterised by** that the sealing surface (26) is a cylindrical shell, and the sealing surface (26) is bordered by a spiral shaped confining ridge (26a).

4. Multifunction drip unit as in any of the claims 1 to 3, **characterised by** that the sealing surface (26) of the cock piece (25) is connected to the drive head (27), and the drive head (27)is placed outside the bordering shell (21) of the flow control unit (20).

5. Multifunction drip unit as in claim 1, **characterised by** that either the drive head (27) and the house (11) or the bordering shell (21) is provided with a scale (32) and the other one is provided with marking (31).
